(19)

Europäisches Patentamt
European Patent Office
Office européen des brevets

(11) **EP 1 286 942 B1**

(12) **EUROPÄISCHE PATENTSCHRIFT**

(45) Veröffentlichungstag und Bekanntmachung des Hinweises auf die Patenterteilung:
**22.04.2009 Patentblatt 2009/17**

(21) Anmeldenummer: **01940393.0**

(22) Anmeldetag: **03.05.2001**

(51) Int Cl.:
**C07C 51/215** (2006.01) **C07C 51/25** (2006.01)
**B01J 23/652** (2006.01) **C07C 53/08** (2006.01)

(86) Internationale Anmeldenummer:
**PCT/EP2001/004987**

(87) Internationale Veröffentlichungsnummer:
**WO 2001/090039 (29.11.2001 Gazette 2001/48)**

(54) **VERFAHREN UND KATALYSATOR ZUR SELEKTIVEN HERSTELLUNG VON ESSIGSÄURE DURCH KATALYTISCHE OXIDATION VON ETHAN UND/ODER ETHYLEN**

METHOD AND CATALYST FOR THE SELECTIVE PRODUCTION OF ACETIC ACID BY CATALYTIC OXIDATION OF ETHANE AND/OR ETHYLENE

PROCEDE ET CATALYSEUR POUR LA PREPARATION SELECTIVE D'ACIDE ACETIQUE PAR OXYDATION CATALYTIQUE D'ETHANE ET/OU D'ETHYLENE

(84) Benannte Vertragsstaaten:
**AT BE CH CY DE DK ES FI FR GB GR IE IT LI LU MC NL PT SE TR**

(30) Priorität: **19.05.2000 DE 10024437**

(43) Veröffentlichungstag der Anmeldung:
**05.03.2003 Patentblatt 2003/10**

(73) Patentinhaber: **Celanese International Corporation**
**Dallas, TX 75381 (US)**

(72) Erfinder:
• **ZEYSS, Sabine**
**61462 Königstein (DE)**

• **DINGERDISSEN, Uwe**
**64342 Seeheim-Jugenheim (DE)**

(74) Vertreter: **Ackermann, Joachim et al**
**Postfach 11 13 26**
**60048 Frankfurt am Main (DE)**

(56) Entgegenhaltungen:
**EP-A- 0 895 809** **DE-A- 19 745 902**
**US-A- 5 049 692**

Bemerkungen:
Die Akte enthält technische Angaben, die nach dem Eingang der Anmeldung eingereicht wurden und die nicht in dieser Patentschrift enthalten sind.

## Beschreibung

Die vorliegende Erfindung betrifft ein Verfahren zur selektiven Herstellung von Essigsäure durch katalytische Gasphasenoxidation von Ethan und/oder Ethylen in Gegenwart eines Molybdän und Palladium enthaltenden Katalysators.

Die oxidative Dehydrierung von Ethan zu Ethylen in der Gasphase, bei Temperaturen > 500°C ist beispielsweise aus US-A-4,250,346, US-A-4,524,236 und US-A-4,568,790 bekannt. Aus US 5,049,692 und EP 0 895 809 ist weiterhin bekannt, dass Niobammoniumsalze zur Herstellung von niobenthaltenden Katalysatoren verwendet werden können. Aus den Dokumente ist aber nicht zu entnehmen, dass die daraus resultierenden Katalysatoren verbesserte Eigenschaften bei der selektiven Herstellung von Essigsäure aus Ethan besitzen.

So beschreibt die US-A-4,250,346 die Verwendung einer Katalysatorzusammensetzung, die die Elemente Molybdän, X und Y im Verhältnis a:b:c enthält zur Umwandlung von Ethan in Ethylen, worin X gleich Cr, Mn, Nb, Ta, Ti, V, und/oder W ist und Y gleich Bi, Ce, Co, Cu, Fe, K, Mg, Ni, P, Pb, Sb, Si, Sn, Tl und/oder U ist und a gleich 1, b gleich 0,05 bis 1 und c gleich 0 bis 2 ist. Der Gesamtwert von c für Co, Ni und/oder Fe muss dabei weniger als 0,5 betragen.

Die Reaktion wird vorzugsweise in Anwesenheit von zugefügtem Wasser durchgeführt. Die offenbarten Katalysatoren können ebenfalls zur Oxidation von Ethan zu Essigsäure verwendet werden, wobei die Effizienz der Umwandlung zu Essigsäure bei ca. 18 %, bei einer Ethan-Umwandlung von 7.5%, liegt.

Die vorstehend genannten Schriften beschäftigen sich hauptsächlich mit der Herstellung von Ethylen, weniger mit der gezielten Herstellung von Essigsäure. Dagegen beschreibt die EP-B-0 294 845 ein Verfahren zur selektiven Herstellung von Essigsäure aus Ethan, Ethylen oder Gemischen davon mit Sauerstoff in Gegenwart eines Katalysatorgemisches, welches mindestens

A.) einen calcinierten Katalysator der Formel $Mo_xV_y$ oder $Mo_xV_yZ_y$, worin Z eines oder mehrere der Metalle Li, Na, Be, Mg, Ca, Sr, Ba, Zn, Cd, Hg, Sc, Y, La, Ce, Al, Tl, Ti, Zr, Hf, Pb, Nb, Ta, As, Sb, Bi, Cr, W, U, Te, Fe, Co und Ni sein kann, und x gleich 0,5 bis 0,9 ist, y gleich 0,1 bis 0,4 ist; und z gleich 0,001 bis 1 ist und

B.) einen Ethylenhydratationskatalysator und/oder Ethylenoxidationskatalysator enthält. Bei der zweiten Katalysatorkomponente B handelt es sich insbesondere um einen Molekularsiebkatalysator oder einen Palladium enthaltenden Oxidationskatalysator.

Bei der Verwendung des beschriebenen Katalysatorgemisches und Einspeisung eines Gasgemisches bestehend aus Ethan, Sauerstoff, Stickstoff und Wasserdampf durch den Katalysator enthaltenden Reaktor beträgt die maximale Selektivität 27% bei einem Ethanumsatz von 7%. Die hohen Umsatzraten von Ethan werden gemäß der EP 0 294 845 nur mit dem beschriebenen Katalysatorgemisch, nicht jedoch mit einem einzigen, die Komponenten A und B enthaltenden Katalysator erreicht.

Ein weiteres Verfahren zur Herstellung eines Produktes, das Ethylen und/oder Essigsäure enthält wird in EP-B-0 407 091 beschrieben. Hierbei werden Ethan und/oder Ethylen und ein molekularen Sauerstoff enthaltendes Gas bei erhöhter Temperatur mit einer Katalysatorzusammensetzung, die die Elemente A, X und Y enthält, in Kontakt gebracht. A ist hierbei $Mo_dRe_eW_f$, X ist Cr, Mn, Nb, Ta, Ti, V und/oder W und Y ist Bi, Ce, Co, Cu, Fe, K, Mg, Ni, P, Pb, Sb, Si, Sn, Tl und/oder U. Die maximalen Selektivitäten, die bei Verwendung des beschriebenen Katalysators bei der Oxidation von Ethan zu Essigsäure erzielt werden konnten, betragen 78%. Als weitere Nebenprodukte werden Kohlendioxid, Kohlenmonoxid und Ethylen gebildet.

DE 19620542 beschreibt ein Verfahren zur selektiven Herstellung von Essigsäure aus einer gasförmigen Einspeisung von Ethan, Ethylen oder Gemischen davon sowie Sauerstoff bei erhöhter Temperatur, dadurch gekennzeichnet, dass die gasförmige Einspeisung mit einem Katalysator zusammengebracht wird, der die Elemente a:b:c:d:e in Kombination mit Sauerstoff enthält: $Mo_aPd_bRe_cX_d,Y_e$, wobei die Symbole X, Y folgende Bedeutung haben: X = Cr, Mn, Nb, B, Ta, Ti, V und/oder W; Y = Bi, Ce, Co, Cu, Te, Fe, Li, K, Na, Rb, Be, Mg, Ca, Sr, Ba, Ni, P, Pb, Sb, Si, Sn, Tl, und/oder U; die Indizes a, b, c, d und e stehen für die Grammatomverhältnisse der entsprechenden Elemente, wobei a = 1, b > 0, c > 0, d = 0,05 bis 2, e = 0 bis 3 ist. In den angegebenen Beispielen werden bei 280°C und 15 bar Ethanumsätze von bis zu 8 %, Essigsäureselektivitäten von bis zu 91 % erreicht.

In DE 19630832 wird ein Verfahren zur selektiven Herstellung von Essigsäure aus einer gasförmigen Einspeisung von Ethan, Ethylen oder Gemischen davon sowie Sauerstoff bei erhöhter Temperatur beschrieben. Die Einspeisung wird dabei mit einem Katalysator zusammengebracht, der die Elemente Mo, Pd, X und Y in Kombination mit Sauerstoff enthält.

X steht dabei für eines oder mehrere der Elemente ausgewählt aus der Gruppe Cr, Mn, Nb, Ta, Ti, V, Te und W, und Y steht für eines oder mehrere der Elemente ausgewählt aus der Gruppe B, Al, Ga, In Pt, Zn, Cd, Bi, Ce, Co, Rh, Ir, Cu, Ag, Au, Fe, Ru, Os, K, Rb, Cs, Mg, Ca, Sr, Ba, Zr, Hf, Ni, P, Pb, Sb, Si, Sn, Tl und U. Die Grammatomverhältnisse für die entsprechenden Elemente werden dabei wie folgt angegeben: a (Mo) = 1; b (Pd) > 0; c (X) > 0; und d (Y)= 0 - 2.

Die in oben genannter Anmeldung beschriebenen Katalysatoren zeigen eine maximale Raum-Zeit-Ausbeute von 149 kg/(hm$^3$) bei einer Essigsäureselektivität von > 60 Mol%. Raum-Zeit-Ausbeuten kennzeichnen die Menge der produzierten Essigsäure pro Zeit und Katalysatorvolumen.

Gegenstand der Erfindung WO 9847850 ist ein Verfahren zur selektiven Herstellung von Essigsäure aus einer gasförmigen Einspeisung aus Ethan, Ethylen oder Gemischen davon sowie Sauerstoff bei erhöhter Temperatur an einem Katalysator, der die Elemente W, X, Y und Z in den Grammatomverhältnissen a:b:c:d in Kombination mit Sauerstoff enthält: $W_aX_bY_cZ_d$, worin X eines oder mehrere Elemente ausgewählt aus der Gruppe Pd, Pt, Ag und/oder Au, Y eines oder mehrere Elemente ausgewählt aus der Gruppe V, Nb, Cr, Mn, Fe, Sn, Sb, Cu, Zn, U, Ni und/oder Bi, Z eines oder mehrere Elemente ausgewählt aus der Gruppe Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As und/oder Te, a = 1, b > 0, c > 0, d = 0 bis 2, sind, sowie der Katalysator selbst.

Bei 250°C, 15 bar und 20 s Verweilzeit wurde bei einem Ethanumsatz von 10 % eine Essigsäureselektivität von 80 % erreicht.

WO 00/14047 betrifft ein Verfahren zur Herstellung von Essigsäure, wobei Ethan und/oder Ethylen mit einem molekularen Sauerstoff-enthaltenden Gas in einem Wirbelschichtreaktor in Gegenwart eines mikrospheroidal fluidisiertem Festkörper-Oxidationskatalysators reagieren, wobei mindestens 90 % der besagten Katalysatorpartikel kleiner als 300 μm sind. Als Katalysator wird eine Verbindung der Zusammensetzung $Mo_aW_bAg_cIr_dX_eY_f$ beschrieben, wobei X für Nb und V, Y für eines der Elemente aus der Gruppe Cr, Mn, Ta, Ti, B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Zr, Hf, Ni, P, Pb, Sb, Si, Sn, Tl, U, Re und Pd stehen; a bis f die Grammverhältnisse der Elemente mit 0 < a ≤ 1, 0 ≤ b < 1 und a + b = 1, 0 < (c + d) ≤ 0,1, 0 < e ≤ 2, und 0 ≤ f ≤ 2 sind. Die angegebenen Beispiele erreichen für die Ethanoxidation zu Essigsäure maximale Raum-Zeit-Ausbeuten an Essigsäure von 354,4 kg/(m$^3$h), für die Ethylenoxidation zu Essigsäure eine Raum-Zeit-Ausbeute an Essigsäure von 258,52 kg/(m$^3$h).

In DE 19745902 wurde gefunden, dass es möglich ist, bei Verwendung eines Katalysators, der die Elemente Molybdän und Palladium und eines oder mehrere Elemente aus der Gruppe Chrom, Mangan, Niob, Tantal, Titan, Vanadium, Tellur und/oder Wolfram enthält, Ethan und/oder Ethylen unter relativ milden Bedingungen, in einfacher Weise mit hoher Selektivität und hervorragenden Raum-Zeit-Ausbeuten zu Essigsäure zu oxidieren.

Diese Erfindung DE 19745902 betrifft somit ein Verfahren zur selektiven Herstellung von Essigsäure aus einer gasförmigen Einspeisung aus Ethan, Ethylen oder Gemischen davon sowie Sauerstoff bei erhöhter Temperatur, wobei die gasförmige Einspeisung mit einem Katalysator zusammengebracht wird, der die Elemente Mo, Pd, X und Y in den Grammatomverhältnissen a:b:c:d in Kombination mit Sauerstoff enthält: $Mo_aPd_bX_cY_d$; und die Symbole X und Y folgende Bedeutung haben: X steht für eines oder mehrere Elemente ausgewählt aus der Gruppe: Cr, Mn, Ta, Ti, V, Te und W, insbesondere V und W; Y steht für eines oder mehrere Elemente ausgewählt aus der Gruppe: B, Al, Ga, In, Pt, Zn, Cd, Bi, Ce, Co, Cu, Rh, Ir, Au, Ag, Fe, Ru, Os, K, Rb, Cs, Mg, Ca, Sr, Ba, Nb, Zr, Hf, Ni, P, Pb, Sb, Si, Sn, Tl und U, insbesondere Nb, Ca, Sb und Li. Die Indizes a, b, c und d stehen für die Grammatomverhältnisse der entsprechenden Elemente, wobei a = 1, b = 0,0001 bis 0,01, c = 0,4 bis 1 und d = 0,005 bis 1 ist. Sofern X und Y für mehrere verschiedene Elemente stehen, können die Indices c und d ebenfalls mehrere unterschiedliche Werte annehmen.

Weiterhin betrifft die genannte Erfindung einen Katalysator zur selektiven Herstellung von Essigsäure enthaltend die Elemente Mo, Pd, X und Y in den Grammatomverhältnissen a:b:c:d in Kombination mit Sauerstoff. Die Grammatomverhältnisse a:b:c:d liegen vorzugsweise in folgenden Bereichen: a= 1; b=0,0001 bis 0,005; c=0,5 bis 0,8 und d=0,01 bis 0,3.

Palladiumgehalte im Katalysator, die über der angegebenen Obergrenze liege führen bei dem in DE 19745902 beschriebenen Verfahren zu einer Begünstigung der Kohlendioxidbildung. Ferner werden höhere Palladiumgehalte allgemein auch deshalb vermieden, da sie den Katalysator unnötig verteuern Dagegen wird bei Palladiumgehalten unterhalb des angegebenen Grenzwertes eine Bevorzugung der Ethylenbildung beobachtet.

Vorzugsweise enthält der in DE 19745902 verwendete Katalysator außer den Elementen Molybdän und Palladium noch Vanadium, Niob, Antimon und Calcium in Kombination mit Sauerstoff. Die Grammatomverhältnisse a:b:c$^1$:d$^1$:d$^2$:d$^3$ der Elemente Mo:Pd:V:Nb:Sb:Ca sind vorzugsweise wie folgt: a (Mo)=1; b (Pd)= 0,0001 bis 0,005, insbesondere 0,0001 bis 0,001; c$^1$ (V)= 0,4 bis 1,0; d$^1$ (Nb)= 0,01 bis 0,2; d$^2$ (Sb)= 0,01 bis 0,3; d$^3$(Ca)= 0,01 bis 0,3.

Die in DE 19745902 unter Beispiel 7 erzielte Raum-Zeit-Ausbeute betrug 470 kg/(hm$^3$) bei 310°C, 15 bar und einer Verweilzeit von 7 s.

WO 00/00284 beschreibt ein Katalysatorsystem auf Basis von MoVNbPd, MoVLaPd oder deren Mischungen für die Herstellung von Essigsäure aus Ethylen. Die angegebenen Beispiele weisen eine maximale Raum-Zeit-Ausbeute von 1291 kg/(m$^3$h) bei einem Ethylenumsatz von 63,43 % und einer Essigsäureselektivität von 78,03 % aus.

Mit den o. g. Patenten wird deutlich, dass bei der Oxidation von Ethylen zwar Essigsäure-Raum-Zeit-Ausbeuten von bis zu 1291 kg/(m$^3$h) erreicht werden können, dass die Raum-Zeit-Ausbeuten an Essigsäure bei der bxidativen Umsetzung von Ethan aufgrund der erschwerten Ethanaktivierung im Vergleich zur Ethylenaktivierung jedoch deutlich unterhalb dieser für die Ethylenoxidation erreichbaren Raum-Zeit-Ausbeute bleiben. In DE-A-197 45 902 wird die bisher größte Essigsäure-Raum-Zeit-Ausbeute von 470 kg/(m$^3$h) für die Oxidation von Ethan beschrieben. Höhere Raum-Zeit-Ausbeuten sind wünschenswert, da hierdurch die Größe der Reaktoren sowie die Menge des im Kreis geführten Gases verringert werden können.

Es besteht daher die Aufgabe, einen Katalysator und ein Verfahren zur Verfügung zu stellen, die es erlauben, Ethan und/oder Ethylen in einfacher Weise, gezielt und mit hoher Selektivität und Raum-Zeit-Ausbeute unter möglichst milden Reaktionsbedingungen zu Essigsäure zu oxidieren.

Die Aufgabe wird durch ein Verfahren gemäß Anspruch 1 und durch die Bereitstellung von Katalysatoren gemäß Anspruch 12 gelöst.

Dazu wird eine modifizierte Methode zur Herstellung von in DE.A-197 45 902 beschriebenen Katalysatoren ähnlicher Zusammensetzung beschrieben, die zu verbesserten katalytischen Eigenschaften der angegebenen Katalysatoren führt. Das besondere an der vorliegende Erfindung ist u. a., dass mit dem beschriebenen Katalysator neben der Oxidation von Ethylen auch die erheblich schwierigere Oxidation von Ethan bei optimierten Reaktionsbedingungen zu hohen Ethanumsätzen, hohen Essigsäureselektivitäten und besonders zu hohen Essigsäure-Raum-Zeit-Ausbeuten im Vergleich zu den genannten Patenten führt.

In DE-A-197 45 902 wurden die Katalysatoren nach dem herkömmlichen Verfahren hergestellt. Hierzu ging man von einer Aufschlämmung, insbesondere einer wässrigen Lösung, die die einzelnen Ausgangskomponenten der Elemente entsprechend ihrer Anteile enthält, aus. Die Ausgangsmaterialien der Einzelkomponenten zur Herstellung des erfindungsgemäßen Katalysators waren neben den Oxiden vorzugsweise in Wasser lösliche Substanzen wie Ammoniumsalze, Nitrate, Sulfate, Halogenide, Hydroxide und Salze organischer Säuren, die durch Erwärmung in die entsprechenden Oxide umgewandelt werden können. Zur Vermischung der Komponenten wurden wässrige Lösungen oder Suspensionen der Metallsalze hergestellt und vermischt. Bei Molybdän wurde aufgrund der kommerziellen Verfügbarkeit empfohlen, als Ausgangsverbindungen die entsprechenden Molybdate, wie z. B. Ammoniummolybdat, einzusetzen. Als Palladiumverbindungen kamen beispielsweise Palladium(II)-chlorid, Palladium(II)-sulfat, Palladium(II)-tetraminnitrat, Palladium(II)-nitrat sowie Palladium(II)-acetylacetonat zum Einsatz.

Die vorliegende Erfindung beschreibt die Herstellung des Katalysators nach einer anderen Methode, unter Verwendung anderer Ausgangsmaterialien als diese in DE-A-197 45 902 beschrieben wurden. So wird anstelle von Nioboxalat ein Niobammoniumcarboxylat, vorzugsweise Niobammoniumoxalat der Zusammensetzung $X_3NbO(C_2O_4)_3 + X_2NbO(OH)(C_2O_4)_2$ mit $X = H^+$ oder $NN_4^+$ (Hersteller: H. C. Starck) verwendet, welches über einen Nb-Gehalt von mindestens 19 Ges.-%, Ammoniak-Gehalten zwischen 0 und 12 Ges.-% sowie typischen Oxalat-Gehalten zwischen 50 und 65 Ges.-% verfügen kann. Überraschenderweise wurde gefunden, dass die Verwendung von Niobammoniumsalz, wie Niobammoniumoxalat als Niobquelle zu besseren katalytischen Eigenschaften führt, was im wesentlichen im Vgl. zu DE-A-197 45 902 auf eine andere Nb-Verteilung im Katalysator zurückgeführt werden kann. Darüber hinaus wird die in DE-A-197 45 902 beschriebene Herstellmethode für die Katalysatoren auch dadurch modifiziert, dass Palladiumacetat in einem Alkohol, insbesondere in Ethanol, und nicht in Aceton gelöst wird. Auch diese Herangehensweise bringt verbesserte katalytische Aktivitäten, die im wesentlichen auf eine verbesserte Verteilung von Pd im Gesamtgemisch zurückzuführen ist.

Die vorliegende Erfindung betrifft somit auch einen niobhaltigen Katalysator des obengenannten Typs der erhältlich ist durch Einsatz eines Niobammoniumcarboxylats, als Niobquelle.

Die erhaltene Reaktionsmischung wird dann 5 Minuten bis 5 Stunden bei 50 bis 100 °C gerührt. Anschließend wird das Wasser entfernt und der verbleibende Katalysator bei einer Temperatur von 50 bis 150°C, insbesondere 80 bis 120°C getrocknet.

Für den Fall, dass der erhaltene Katalysator anschließend noch einem Calcinierungsprozess unterworfen wird, empfiehlt es sich, den getrockneten und pulverisierten Katalysator bei einer Temperatur im Bereich von 100°C bis 800°C, insbesondere 200 bis 500°C in Gegenwart von Stickstoff, Sauerstoff oder eines sauerstoffhaltigen Gases zu calcinieren. Die Calcinierung kann in einem Muffelofen, besser jedoch in einem Drehrohrofen, in dem der Katalysator kontinuierlich mit dem entsprechenden Gas durchströmt wird, was wiederum zu einer verbesserten Homogenität des Katalysators im Vgl. zu den in DE 19745902 beschriebenen Katalysatoren führt, durchgeführt werden. Die Zeitdauer der Calcinierung beträgt 2 bis 24 Stunden.

Der Katalysator kann ohne ein entsprechendes Trägermaterial eingesetzt werden oder mit einem solchen gemischt oder auf ein solches aufgebracht werden. Geeignet sind übliche Trägermaterialien, wie z. B. poröses Siliziumdioxid, geglühtes Siliziumdioxid, Kieselgur, Kieselgel, poröses oder nicht poröses Aluminiumoxid, Titandioxid, Zirkoniumdioxid, Thoriumdioxid, Lanthanoxid, Magnesiumoxid, Calciumoxid, Bariumoxid. Zinnoxid, Cerdioxid, Zinkoxid. Boroxid, Bornitrid, Borcarbid, Borphosphat, Zirkoniumphosphat, Aluminiumsilikat, Siliziumnitrid oder Siliziumcarbid aber auch Glas-, Kohlefaser-, Metalloxid- oder Metallnetze oder entsprechende Monolithe.

Bevorzugte Trägermaterialien haben eine Oberfläche von weniger als 100 $m^2$/g. Bevorzugte Trägermaterialien sind Siliziumdioxid und Aluminiumoxid mit geringer spezifischer Oberfläche. Der Katalysator kann nach der Formgebung als regelmäßig oder unregelmäßig geformter Trägerkörper oder aber in Pulverform als heterogener Oxidationskatalysator eingesetzt werden.

Die Reaktion kann in der Wirbelschicht oder in einem Festbettreaktor durchgeführt werden. Für den Einsatz in einer Wirbelschicht wird der Katalysator durch gängige Verfahren, z. B. der Agglomeration, so präpariert, dass eine bevorzugte Korngrößenverteilung im Bereich von 10 bis 200 μm erzielt werden kann.

Die gasförmige Einspeisung enthält Ethan und/oder Ethylen, welche als reine Gase oder in Mischung mit einem oder mehreren anderen Gasen dem Reaktor zugeführt werden. Als solche zusätzlichen oder Trägergase kommen beispielsweise Stickstoff, Methan, Kohlenmonoxid, Kohlendioxid, Luft und/oder Wasserdampf in Frage. Das molekularen Sauerstoff enthaltende Gas kann Luft oder ein an molekularen Sauerstoff reicheres oder ärmeres Gas als Luft, z. B. Sauerstoff, sein. Der Anteil des Wasserdampfes kann im Bereich von 0 bis 50 Vol% liegen. Höhere Wasserdampfkonzentrationen würden die Aufarbeitung der anfallenden wässrigen Essigsäure aus verfahrenstechnischen Gründen unnötig verteuern. Aus diesem Grunde wurde die Wasserdampfkonzentration im Feed in den Beispielen der vorliegenden Erfindung im Vergleich zu den in DE 19745902 angegebenen Beispielen weiter gesenkt, was zu einer bedeutenden Kosteneinsparung in der Essigsäureaufarbeitung führen würde. Das Verhältnis von Ethan/Ethylen zu Sauerstoff liegt günstigerweise im Bereich zwischen 1:1 und 10:1, vorzugsweise 2:1 und 8:1. Höhere Sauerstoffgehalte sind bevorzugt, da der erreichbare Ethanumsatz und somit die Ausbeute an Essigsäure höher sind. Bevorzugt ist die Zugabe von Sauerstoff oder des molekularen Sauerstoff enthaltenen Gases in einem Konzentrationsbereich außerhalb der Explosionsgrenzen unter Reaktionsbedingungen, da hierdurch die Durchführung des Verfahrens vereinfacht wird. Allerdings ist es auch möglich, das Ethan/Ethylen zu Sauerstoffverhältnis innerhalb der Explosionsgrenzen einzustellen.

Die Reaktion wird bei Temperaturen zwischen 200 und 500°C, bevorzugt 200 bis 400°C durchgeführt. Der Druck kann atmosphärisch oder superatmosphärisch sein, z.B. im Bereich zwischen 1 und 50 bar, bevorzugt 1 bis 30 bar.

Die Reaktion kann in einem Festbett- oder Wirbelschichtreaktor durchgeführt werden. Zweckmäßigerweise wird Ethan zunächst mit den inerten Gasen wie Stickstoff oder Wasserdampf gemischt, bevor Sauerstoff oder das molekularen Sauerstoff enthaltende Gas zugeführt wird. Die vermischten Gase werden bevorzugt in einer Vorheizzone auf die Reaktionstemperatur vorgeheizt, bevor das Gasgemisch mit dem Katalysator in Kontakt gebracht wird. Aus dem Reaktorabgas wird Essigsäure durch Kondensation abgetrennt. Die übrigen Gase werden an den Reaktoreingang zurückgeführt, wo Sauerstoff oder das molekularen Sauerstoff enthaltende Gas sowie Ethan und/oder Ethylen zudosiert wird.

Bei einem Vergleich der erfindungsgemäßen Katalysatoren mit denen im Stand der Technik bekannten findet man, dass mit den vorliegenden Katalysatoren unter gleichen Reaktionsbedingungen (Reaktionseingangsgas, Druck, Temperatur) höhere Raum-Zeit-Ausbeuten und Essigsäureselektivitäten erreicht werden.

Bei Verwendung des erfindungsgemäßen Katalysators liegt die Selektivität bei der Oxidation von Ethan und/ oder Ethylen zu Essigsäure bei ≥ 70 Mol%, vorzugsweise ≥ 80 Mol%, insbesondere ≥ 90 Mol%, und die Raum-Zeit-Ausbeute bei > 470 kg/(hm$^3$), insbesondere > 500 kg/(hm$^3$) vorzugsweise > 550 kg/(hm$^3$), so dass mit dem erfindungsgemäßen Verfahren im Vergleich mit dem Stand der Technik eine Erhöhung der Essigsäureausbeuten, bei gleichzeitiger Verminderung des Anfalls von ungewünschten Nebenprodukten, auf einfache Weise erzielt werden kann.

Beispiele

Die im Beispiel aufgeführte Katalysatorzusammensetzung ist in relativen Atomverhältnissen angegeben.

**Katalysatorpräparation:**

Katalysator (I):

Ein Katalysator mit folgender Zusammensetzung wurde hergestellt: $Mo_{1,000}Pd_{0.00075}V_{0,55}Nb_{0,09}Sb_{0,01}Ca_{0,01}O_x$

Lösung 1:

80 g Ammoniummolybdat $(NH_4)_6Mo_7O_4$ x $4H_2O$ (Riedel-de Haen) in 400 ml Wasser.

Lösung 2:

29,4 g Ammoniummetavanadat $NH_4VO_3$ (Riedel-de Haen) in 400 ml Wasser.

Lösung 3:

> 19,01 g Nioboammoniumoxalat (H. C. Starck),
> 1,92 g Antimonoxalat $Sb_2(C_2O_4)_3$ (Pfaltz & Bauer),
> 1,34 g Calciumnitrat $Ca(NO_3)_2$ x $4H_2O$ (Riedel-de Haen) in 200 ml Wasser.

Lösung 4:

> 0,078 g Palladium(II)acetat $(CH_3CO_2)_2Pd$ (Aldrich) in 200 ml Ethanol.

Die wässrigen Lösungen 1 bis 3 werden separat bei 70°C für 15 Minuten gerührt. Dann wird die dritte Lösung zur zweiten hinzugegeben. Die vereinigten Mischungen werden bei 70°C für 15 Minuten gerührt bevor diese zur ersten gegeben werden. Hiernach gibt man Lösung 4 zu. Die erhaltene Mischung wird bei 70°C für 15 Minuten gerührt und anschließend auf ein Volumen von 800 ml eingedampft. Das Gemisch wird sprühgetrocknet und in statischer Luft bei 120°C für 2 Stunden und bei 300°C für 5 Stunden calciniert. Der Katalysator wird hiernach leicht gemörsert und zu Tabletten gepresst. Diese werden über ein Sieb zerstoßen, um eine Siebfraktion zwischen 0,35 und 0,7 mm zu erhalten.

**Methode zur Katalysatoraustestung:**

5 oder 10 ml des Katalysators wurden in einen Stahlreaktor mit 14 mm Innendurchmesser geladen. Der Katalysator wurde unter einem Luftstrom auf 250°C aufgeheizt. Anschließend wurde der Druck mittels eines Vordruckreglers eingestellt. Das gewünschte Ethan:Sauerstoff:Stickstoff-Gemisch wurde mit Wasser in eine Verdampferzone eindosiert, wo Wasser verdampfte und mit den Gasen vermischt wurde. Die Reaktionstemperatur wurde mit einem Thermoelement in der Katalysatorschüttung gemessen. Das Reaktionsgas wurde on-line gaschromatographisch analysiert.
In den Beispielen sind die folgende Begriffe definiert als:

$$\text{Ethanumsatz (\%)} =$$

$$([CO]/2 + [CO_2]/2 + [C_2H_4] + [CH_3COOH]) / ([CO]/2 + [CO_2]/2 + [C_2H_4] + [C_2H_6] + [CH_3COOH]) * 100$$

$$\text{Ethylenselektivität (\%)} =$$

$$([C_2H_4]) / ([CO]/2 + [CO_2]/2 + [C_2H_4] + [CH_3COOH]) * 100$$

$$\text{Essigsäureselektivität (\%)} =$$

$$([CH_3COOH]) / ([CO]/2 + [CO_2]/2 + [C_2H_4] + [CH_3COOH]) * 100$$

worin

> [ ] = Konzentrationen in mol% und
> $[C_2H_6]$ = Konzentration des nicht umgesetzten Ethans bedeutet.

Die Verweilzeit ist definiert als:

$\vartheta$ (s) = Schüttvolumen des Katalysators (ml) / Volumenstrom des Gases durch den Reaktor bezogen auf die Reaktionsbedingungen (ml/s),
RZA steht für Raum-Zeit-Ausbeute in kg Essigsäure pro Stunde und m$^3$ Katalysator.

Reaktionsdurchführung:

Das Verhältnis Ethan/Sauerstoff/Stickstoff im Reaktionsgas betrug 5/1/4. Der Anteil von Wasserdampf im Reaktionsgemisch wurde auf Werte kleiner/gleich 20 % eingestellt. Da die Raum-Zeit-Ausbeute vom Reaktionsdruck abhängig ist, wurden aus Gründen der Vergleichbarkeit alle Versuchsbeispiele bei 15 bar durchgeführt. Reaktionsbedingungen und Ergebnisse sind in Tabelle 1 zusammengefasst.

**Table 1**

| Ergebnisse katalytischer Untersuchungen der Ethanoxidation zu Essigsäure an Katalysator (I) | | | | | | | | | | | |
|---|---|---|---|---|---|---|---|---|---|---|---|
| | **Reaktionsbedingungen** | | | | | | **Ergebnisse** | | | | |
| | | | Reakitinsgaszusammensetzung | | | | Umsatz | Selektivität | | | Raum-Zeit-Ausbeute |
| Vers-Nr | T [°C] | $\vartheta$ [s] | $V(C_2H_6)$ [ml/s] | $V(O_2)$ [ml/s] | $V(N_2)$ [ml/s] | $V(H_2O)$ [g/h] | $X(C_2H_6)$ [%] | S (HOAc) [%] | S $(C_2H_4)$ [%] | S $(CO+CO_2)$ [%] | RZA (HOAc) [kg/(hm$^3$)] |
| 1 | 280 | 14.8 | 1.0 | 0.2 | 0.8 | 1.4 | 13.3 | 91.5 | 0.7 | 7.8 | 235 |
| 2 | 280 | 7.4 | 2.0 | 0.4 | 1.6 | 2.9 | 10.5 | 90.4 | 3.5 | 6.0 | 362 |
| 3 | 300 | 7.1 | 2.0 | 0.4 | 1.6 | 2.9 | 13.2 | 89.0 | 2.0 | 9.0 | 447 |
| 4 | 300 | 4.8 | 3.0 | 0.6 | 2.4 | 4.3 | 11.3 | 87.2 | 5.5 | 7.3 | 564 |
| 5 | 300 | 4.1 | 3.5 | 0.7 | 2.8 | 5.0 | 10.2 | 86.2 | 7.4 | 6.4 | 584 |
| 6 | 300 | 3.7 | 4.0 | 0.8 | 3.2 | 5.0 | 9.9 | 84.1 | 9.2 | 6.6 | 630 |

Tabelle 1 zeigt, dass die Erniedrigung der Verweilzeit bei gleicher Reaktionsgaszusammensetzung und bei einer Reaktionstemperatur von 280°C zu einer vernachlässigbaren Umsatzabnahme, zu gleichbleibender Essigsäureselektivität, jedoch zu einer anderthalbfach erhöhten Raum-Zeit-Ausbeute führt (vgl. Versuche 1 und 2). Weitere Erniedrigung der Verweilzeit bei 300°C (vgl. Versuche 3 und 4) führt bei vergleichbaren Umsätzen und Selektivitäten zu einer weiteren Erhöhung der Raum-Zeit-Ausbeute. Im Vergleich zu Versuch 4 wird in den Versuchen 5 und 6 neben weiterer Verringerung der Verweilzeit auch der Wassergehalt im Eduktgasstrom reduziert. Bei vergleichbaren Umsätzen und Essigsäureselektivitäten konnte die Raum-Zeit-Ausbeute weiter auf 630 kg/(hm$^3$) erhöht werden. Besonders hervorzuheben ist hierbei auch, dass die marginale Erniedrigung der Essigsäureselektivität im Vergleich zu den Versuchen 1 bis 4 nicht auf die vermehrte Bildung von Totaloxidationsprodukten zurückzuführen ist, sondern dass aufgrund des geringeren Wasseranteils im Feed neben Essigsäure bevorzugt Ethylen gebildet wird, welches als Wertprodukt im Kreis gefahren und zu Essigsäure weiter oxidiert werden kann.

## Patentansprüche

1. Kontinuierliches Verfahren zur selektiven Herstellung von Essigsäure aus einer gasförmigen Einspeisung von Ethan, Ethylen oder Gemischen davon sowie Sauerstoff bei erhöhter Temperatur, worin die gasförmige Einspeisung mit einem Katalysator zusammengebracht wird, der die Elemente Mo, Pd, X und Y in den Grammatomverhältnissen a: b:c:d in Kombination mit Sauerstoff enthält

$$Mo_aPd_bX_cY_d \qquad (I)$$

wobei die Symbole X und Y folgende Bedeutung haben

X steht für eines oder mehrere der Elemente ausgewählt aus der Gruppe Cr, Mn, Ta, Ti, V, Te und W;
Y steht für eines oder mehrere der Elemente ausgewählt aus der Gruppe B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl und U; wobei Y zumindest Nb enthalten muss;
die Indices a, b, c, und d stehen für die Grammatomverhältnisse der entsprechenden Elemente, wobei

a=1;
b= 0,0001 bis 0.01;
c= 0,4 bis 1; und
d= 0,005 bis 1 ist,

und worin die Verweilzeiten und die Zusammensetzung der gasförmigen Einspeisung so gewählt werden, dass die Raum-Zeit-Ausbeute bei der Oxidation zu Essigsäure bei > 470 kg/(hm$^3$) liegt, mit der Massgabe, dass der Katalysator durch den Einsatz eines Niobammoniumcarboxylates als Niobquelle erhalten worden ist.

2. Verfahren nach Anspruch 1, **dadurch gekennzeichnet, dass** Niobammoniumoxalat als Niobquelle zum Einsatz kommt.

3. Verfahren nach mindestens einem der Ansprüche 1 und 2, **dadurch gekennzeichnet, dass** die Temperatur im Bereich von 200 bis 500°C liegt.

4. Verfahren nach mindestens einem der Ansprüche 1 bis 3, **dadurch gekennzeichnet, dass** der Druck im Reaktor im Bereich von 1 bis 50 bar liegt.

5. Verfahren nach mindestens einem der Ansprüche 1 bis 4, **dadurch gekennzeichnet, dass** b im Bereich von 0,0001 bis 0,001 liegt.

6. Verfahren nach mindestens einem der Ansprüche 1 bis 5, **dadurch gekennzeichnet, dass** dem Reaktor Ethan gemischt mit mindestens einem weiteren Gas zugeführt wird.

7. Verfahren nach Anspruch 6, **dadurch gekennzeichnet, dass** das als weiteres Gas Stickstoff, Sauerstoff, Methan, Kohlenmonoxid, Kohlendioxid, Ethylen und /oder Wasserdampf zugeführt wird.

8. Verfahren nach mindestens einem der Ansprüche 1 bis 7, **dadurch gekennzeichnet, dass** der Katalysator mit einem Trägermaterial gemischt oder auf einem Trägermaterial fixiert ist.

9. Verfahren nach mindestens einem der Ansprüche 1 bis 8**, dadurch gekennzeichnet, dass** die Selektivität der Oxidationsreaktion von Ethan und/oder Ethylen zu Essigsäure bei Werten ≥ 70 Mol% liegt.

10. Verfahren nach mindestens einem der Ansprüche 1 bis 9, **dadurch gekennzeichnet, dass** ein Katalysator eingesetzt wird, bei dem das Pd in Form einer alkoholischen Lösung von Palladiumacetat eingebracht worden ist.

11. Verfahren nach mindestens einem der Ansprüche 1 bis 10, **dadurch gekennzeichnet, dass** ein Katalysator eingesetzt wird, bei dem das Nb durch Niobammoniumoxalat als Niobquelle und das Pd in Form einer alkoholischen Lösung von Palladiumacetat eingebracht worden ist.

12. Katalysator zur selektiven Oxidation von Ethan, Ethylen oder Gemischen davon sowie Sauerstoff, enthaltend die Elemente Mo, Pd, X und Y in den Grammatomverhältnissen a:b:c:d in Kombination mit Sauerstoff

$$Mo_aPd_bX_cY_d \qquad (I)$$

wobei die Symbole X und Y folgende Bedeutung haben

X steht für eines oder mehrere der Elemente ausgewählt aus der Gruppe: Cr, Mn, Ta, Ti, V, Te und W;
Y steht für eines oder mehrere der Elemente ausgewählt aus der Gruppe: B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl und U; wobei Y zumindest Nb enthalten muss;
die Indizes a, b, c und d stehen für die Grammatomverhältnisse der entsprechenden Elemente, wobei

a=1;
b=0,0001 bis 0,01;
c= 0,4 bis 1 und
d= 0,005 bis 1 ist, und

die Raum-Zeit-Ausbeute bei der Oxidationsreaktion bei > 470 kg/(hm$^3$) liegt, mit der Massgabe, dass der Katalysator durch den Einsatz eines Niobammoniumcarboxylates als Niobquelle erhalten worden ist.

13. Katalysator nach Anspruch 12, wobei Niobammoniumoxalat als Niobquelle zum Einsatz kommt.

14. Katalysator nach Anspruch 12, wobei das Pd in Form einer alkoholischen Lösung von Palladiumacetat eingebracht worden ist.

**Claims**

1. A continuous process for the selective preparation of acetic acid from a gaseous feed of ethane, ethylene or mixtures thereof and oxygen at elevated temperature, in which the gaseous feed is brought together with a catlayst comprising the elements Mo, Pd, X and Y in gram-atom ratios a:b:c:d in combination with oxygen

$$Mo_aPd_bX_cY_d \qquad (I)$$

where the symbols X and Y are defined below:

X is one or more elements selected from the group consisting of Cr, Mn, Ta, Ti, V, Te and W;
Y is one or more elements selected from the group consisting of B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, TI und U; wherein Y comprises at least Nb;
the indices a, b, c and d are the gram-atom ratios of the corresponding elements, where

a = 1;
b = from 0,0001 to 0,01;
c = from 0,4 to 1; and

d = from 0,005 to 1,

and in which the residence times and the composition of the gaseous feed are selected in such a way that the space-time yield in the oxidation to acetic acid is >470 kg/(hm$^3$), with the proviso that the catalyst has been obtained by the use of a niobium ammonium carboxylate as niobium source.

2. The process as claimed in claim 1, wherein niobium ammonium oxalate is used as niobium source.

3. The process as claimed in at least one of claims 1 and 2, wherein the temperature is in the range from 200 to 500°C.

4. The process as claimed in at least one of claims 1 to 3, wherein the pressure in the reactor is in the range from 1 to 50 bar.

5. The process as claimed in at least one of claims 1 to 4, wherein b is in the range from 0.0001 to 0.001.

6. The process as claimed in at least one of claims 1 to 5, wherein ethane mixed with at least one further gas is fed to the reactor.

7. The process as claimed in claim 6, wherein the further gas fed in is nitrogen, oxygen, methane, carbon monoxide, carbon dioxide, ethylene and/or steam.

8. The process as claimed in at least one of claims 1 to 7, wherein the catalyst is mixed with a support material or immobilized on a support material.

9. The process as claimed in at least one of claims 1 to 8, wherein the selectivity of the oxidation reaction of ethane and/or ethylene to acetic acid is $\geq$ 70 Mol%.

10. The process as claimed in at least one of claims 1 to 9, wherein the catalyst comprises Pd introduced in the form of an alcoholic solution of palladium acetate.

11. The process as claimed in at least one of claims 1 to 10, wherein a catalyst is used, where the Nb has been introduced with niobium oxalate as niobium source and the Pd has been introduced in form of an alcoholic solution of palladium acetate.

12. A catalyst for the selective oxidation of ethane, ethylene or mixtures thereof and oxygen, comprising the elements Mo, Pd, X and Y in gram-atom ratios a:b:c:d in combination with oxygen

$$Mo_aPd_bX_cY_d \qquad (I)$$

where the symbols X and Y are defined as below:

X is one or more elements selected from the group consisting of Cr, Mn, Ta, Ti, V, Te and W;
Y is one of more elements selected from the group consisting of B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl und U; wherein Y comprises at least Nb;
the indices a, b, c and d are the gram-atom ratios of the corresponding elements, where

a = 1;
b = from 0,0001 to 0,01;
c = from 0,4 to 1 and
d = from 0,005 to 1, and

the space-time yield in the oxidation reaction is > 470 kg/(hm$^3$), with the proviso that the catalyst has been obtained by the use of a niobium ammonium carboxylate as niobium source.

13. The catalyst as claimed in claim 12, where niobium ammonium oxalate is used as niobium source.

14. The catalyst as claimed in claim 12, where the Pd has been introduced in the form of an alcoholic solution of palladium acetate.

**Revendications**

1. Procédé continu pour la préparation sélective d'acide acétique à partir d'une alimentation gazeuse d'éthane, d'éthylène ou de mélanges de ceux-ci, de même que d'oxygène à température accrue, dans lequel l'alimentation gazeuse est mise en contact avec un catalyseur qui contient les éléments Mo, Pd, X et Y dans les rapports d'atome-gramme a : b : c : d en combinaison avec de l'oxygène

$$Mo_aPd_bX_cY_d \qquad (I)$$

où les symboles X et Y ont les significations suivantes :

X représente un ou plusieurs des éléments choisis parmi le groupe Cr, Mn, Ta, Ti, V, Te et W ;
Y représente un ou plusieurs des éléments choisis parmi le groupe B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl et U, sachant que Y doit au moins contenir Nb ;
les indices a, b, c, d représentent les rapports d'atome-gramme des éléments correspondants, sachant que
a=1
b = 0,0001 à 0,01
c = 0,4 à 1 et
d = 0,005 à 1
et dans lequel le temps de séjour et la composition de l'alimentation gazeuse sont choisis de telle sorte que le rendement espace-temps lors de l'oxydation en acide acétique se trouve être >470kg/(h.m3), dans la mesure où le catalyseur a été obtenu par l'utilisation d'un carboxylate de niobium et d'ammonium comme source de niobium.

2. Procédé selon la revendication 1**, caractérisé en ce que** de l'oxalate de niobium et d'ammonium est utilisé comme source de niobium.

3. Procédé selon l'une au moins des revendications 1 et 2, **caractérisé en ce que** la témpérature est comprise dans l'intervalle de 200 à 500°C.

4. Procédé selon l'une au moins des revendications 1 à 3, **caractérisé en ce que** la pression dans le réacteur est comprise dans l'intervalle de 1 à 50 bar.

5. Procédé selon l'une au moins des revendications 1 à 4, **caractérisé en ce que** b est compris dans l'intervalle de 0,0001 à 0,001.

6. Procédé selon l'une au moins des revendications 1 à 5, **caractérisé en ce que** l'on achemine dans le réacteur l'éthane mélangé à au moins un autre gaz.

7. Procédé selon la revendication 6, **caractérisé en ce qu'**en tant qu'autre gaz, on achemine de l'azote, de l'oxygène, du méthane, du monoxyde de carbone, du dioxyde de carbone, de l'éthylène et/ou de la vapeur d'eau.

8. Procédé selon l'une au moins des revendications 1 à 7, **caractérisé en ce que** le catalyseur est mélangé à un matériau support ou est fixé sur un matériau support.

9. Procédé selon l'une au moins des revendications 1 à 8, **caractérisé en ce que** la sélectivité de la réaction d'oxydation de l'éthane et/ou de l'éthylène en acide acétique est située à des valeurs $\geq 70$ % en mole.

10. Procédé selon l'une au moins des revendications 1 à 9, **caractérisé en ce que** l'on utilise un catalyseur dans lequel le Pd a été introduit sous la forme d'une solution alcoolique d'acétate de palladium.

11. Procédé selon l'une au moins des revendications 1 à 10, **caractérisé en ce que** l'on utilise un catalyseur dans lequel le Nb a été introduit sous la forme d'oxalate d'ammonium et de niobium comme source de niobium et le Pd sous la forme d'une solution alcoolique d'acétate de palladium.

**12.** Catalyseur pour l'oxydation sélective d'éthane, d'éthylène ou de mélanges de ceux-ci, de même que d'oxygène, contenant les éléments Mo, Pd, X et Y dans les rapports d'atome-gramme a : b : c : d en combinaison avec de l'oxygène

$$Mo_aPd_bX_cY_d \qquad (I)$$

où les symboles X et Y ont les significations suivantes :

X représente un ou plusieurs des éléments choisis parmi le groupe Cr, Mn, Ta, Ti, V, Te et W ;
Y représente un ou plusieurs des éléments choisis parmi le groupe B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl et U, sachant que Y doit au moins contenir Nb ;
les indices a, b, c, d représentent les rapports d'atome-gramme des éléments correspondants, sachant que a=1
b = 0,0001 à 0,01
c = 0,4 à 1 et
d = 0,005 à 1 et
le rendement espace-temps lors de la réaction d'oxydation se trouve être >470kg/(h.m$^3$), dans la mesure où le catalyseur a été obtenu par l'utilisation d'un carboxylate de niobium et d'ammonium comme source de niobium.

**13.** Catalyseur selon la revendication 12, **caractérisé en ce que** de l'oxalate de niobium et d'ammonium est mis en oeuvre comme source de niobium.

**14.** Catalyseur selon la revendication 12, **caractérisé en ce que** le Pd a été introduit sous la forme d'une solution alcoolique d'acétate de palladium.

**EP 1 286 942 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4250346 A
- US 4524236 A
- US 4568790 A
- US 5049692 A
- EP 0895809 A
- EP 0294845 B
- EP 0294845 A
- EP 0407091 B
- DE 19620542

- DE 19630832
- WO 9847850 A
- WO 0014047 A
- DE 19745902
- WO 0000284 A
- DE 19745902 A